# FASCICULE DE BREVET EUROPEEN

(11) **EP 1 807 114 B1**
(45) Date de publication et mention de la délivrance du brevet: **06.08.2008**
(21) Numéro de dépôt: 05815499.8
(22) Date de dépôt: 19.10.2005
(51) Int. Cl.: A61K 47/36

(54) **COMPOSITION BIOADHESIVE A LIBERATION PROGRAMMEE**
BIOADHÄSIVE ZUSAMMENSETZUNG MIT PROGRAMMIERTER FREISETZUNG
BIOADHESIVE COMPOSITION WITH PROGRAMMED RELEASE

(30) Priorité: 20.10.2004 FR 0411156
(43) Date de publication de la demande: 18.07.2007
(73) Titulaire: Interpharm Development SA, CH-1020 RENENS/LAUSANNE (CH)
(72) Inventeur: PARIS, Laurence, F-03390 Montmarault (FR)
(74) Mandataire: Thurgood, Alexander John
(86) Numéro de dépôt international: PCT/FR2005/050869
(87) Numéro de publication internationale: WO 2006/043005

(56) Documents cités:
- EP-A- 0 539 627
- WO-A-01/70271
- WO-A-02/40056
- WO-A-98/19663
- FR-A- 2 542 616
- FR-A- 2 848 473
- US-A- 6 159 491
- DATABASE CA [Online] CHEMICAL ABSTRACTS SERVICE, COLUMBUS, OHIO, US; 27 mai 2004 (2004-05-27), KUDO, YUMIO ET AL: "Oral gel preparations for treatment of Candida infection in mouth and esophagus" XP002350123 extrait de STN Database accession no. 2004:433033 & JP 2004 149528 A2 (MOCHIDA PHARMACEUTICAL CO., LTD., JAPAN) 27 mai 2004 (2004-05-27)

## Description

La présente invention a trait au domaine pharmaceutique, cosmétique et nutraceutique et plus particulièrement à celui des systèmes bio-adhésifs à libération programmée des principes actifs dans le corps humain.

Le corps humain est constitué d'un certain nombre de cavités accessibles de l'extérieur et recouvertes de muqueuses. Ces muqueuses sont le siège d'un certain nombre de maladies ou d'infections locales. C'est le cas de :
- la muqueuse buccale qui s'étale de la bouche à l'oesophage en passant par la gorge ;
- la muqueuse nasale qui s'étale du nez à l'arrière gorge
- la muqueuse rectale couvrant la partie distale du gros intestin
- la muqueuse vaginale
- la muqueuse oculaire appelée la cornée.

Ces muqueuses ont la particularité d'être humidifiées en permanence par des liquides spécifiques de la muqueuse en cause.

Ces sécrétions ont deux rôles majeurs au niveau de la muqueuse :
- un rôle de protection de cette dernière vis-à-vis des agressions extérieures
- un rôle de lubrifiant.

C'est ainsi dans le cas du liquide lacrymal, de la salive et des sécrétions vaginales.

L'accessibilité des ces muqueuses par l'extérieur permet de ce fait un traitement dit local si nécessaire.

En effet, un certain nombre de produits existent pour traiter des infections locales tels que :
- des comprimés à sucer pour le traitements des aphtes, des stomatites, gingivites, glossites, etc....
- des gels et crèmes pour des traitements buccaux, vaginaux, rectaux (hémorroïdes), oculaires,
- des liquides pour la voie nasale (solutés nasales) et pour la voie oculaire (collyres).

Cependant ces produits ont une durée d'action très courte sur le plan thérapeutique en raison d'un «lessivage» permanent de ces muqueuses par les sécrétions éliminant ainsi le principe actif déposé *in situ.*

Ceci impose donc des administrations locales répétées qui dans certains cas ne sont pas très agréables pour le patient. A titre d'exemple nous pouvons citer les administrations répétées de collyre.

De plus, pour assurer la rémission de l'infection, le traitement local est généralement associé à un traitement systémique.

On entend par «traitement systémique» l'apport par voie sanguine d'un principe actif au sein de tout l'organisme. Cet apport peut se faire par administration directe dans la circulation sanguine (administration intraveineuse) ou par administration orale.

A titre d'exemple de traitement local associé à un traitement systémique, nous pouvons citer le cas de l'acyclovir, principe actif utilisé dans le traitement de l'herpès.

L'herpès est dû à un virus qui se développe aussi bien au niveau buccal, vaginal et oculaire.

Cette affection est traitée localement par des crèmes. En fonction de la gravité de l'atteinte, le traitement local est associé à un traitement systémique. C'est ainsi que pour un herpès oculaire, le traitement local nécessite 5 applications par jour dans le cul-de-sac conjonctival de l'oeil. La posologie dans le cas d'un herpès vaginal est identique. Dans les cas sévères de ces deux atteintes, une administration orale (administration systémique) est associée au traitement local.

Ainsi il apparaît nécessaire, pour le confort du malade, d'avoir des systèmes qui permettent de maintenir une durée d'action locale plus importante que de simples crèmes, afin de réduire le nombre d'applications par jour.

D'une façon générale tout système qui permet de prolonger l'action du principe actif dans l'organisme est appelé «forme à libération prolongée ou programmée».

A ce jour de tels systèmes ont été en majorité développés pour la voie orale. Ce sont généralement des comprimés ou des gélules contenant des microgranules. Ces formes à libération prolongée ou dites programmées sont nombreuses et appartiennent à différentes catégories en fonction des excipients utilisés pour ralentir la libération des principes actifs. Ce sont :
- les formes matricielles. Ce sont généralement des comprimés à partir desquels le principe actif est libéré soit :
   - par érosion du support
   - par diffusion à travers le réseau formé par les ingrédients du comprimé
- les formes pelliculées. Ce sont des comprimés classiques ou des microgranules, ayant subit un enrobage à l'aide de substances ayant des propriétés particulières permettant une libération lente à travers la membrane formée.

De très nombreux brevets ont été pris dans ce domaine et sont liés aux principes actifs mis en oeuvre dans cette forme.

En dehors de la voie orale, peu de systèmes à libération prolongée ont été développés.

Pour la voie intraveineuse, il n'existe pas ce genre de systèmes.

Dans le cas de la voie intramusculaire, la prolongation de l'action est obtenue par injection d'une suspension de particules destinées à se solubiliser progressivement dans l'organisme. L'exemple le plus connu est celui de l'insuline : injection intramusculaire de particules d'insuline pour une action de 24 heures minimum.

Un deuxième exemple est celui de l'administration d'implants dans le cas de l'hormonothérapie : dépôts de petits comprimés à base d'hormones (progestatifs) sous la peau qui sont destinés à se dissoudre lentement.

Concernant la voie cutanée un système à libération prolongée a été développé pour une administration systémique de principes actifs : ce sont les systèmes transdermiques dits «patch».

L'exemple le plus connu est le patch à la nicotine pour le sevrage des fumeurs.

Sur le plan local très peu de choses existent.

La voie qui a fait l'objet d'une grande attention dans ce domaine, est la voie oculaire.

Un certain nombre de produits ont été développés tels que :
- des gels solubles qui ralentissent la libération du principe actif en raison de leur viscosité élevée. Cette viscosité prolonge le temps de contact du principe actif au niveau de la cornée et son temps de présence dans le cul-de-sac conjonctival. Cette intensification de l'action est due au ralentissement du processus d'élimination au niveau du canal lacrymal, grâce à la viscosité du produit.
   Ces gels sont généralement à base de molécules polymériques hydrosolubles telles que l'alcool polyvinylique, des dérivés cellulosiques et des dérivés acryliques.
   A titre d'exemple peuvent être cités les travaux de :
   - WANG et HAMMARLUNG (1) sur des gels d'alcool polyvinylique et d'hydroxypropylméthylcellulose contenant de l'hypobromite d'homatropine ayant un effet myotique
   - HAAS et Coll. (2) sur des gels de méthylcellulose contenant de la pilocarpine
   - GOLBERG et Coll. (3) ainsi que ceux de MANDELL et Coll. (4) et MARCH et Coll. (5) sur des gels acryliques de haute viscosité contenant de chlorhydrate de pilocarpine
   - SCHOENWALD et Coll. (6) sur des gels de Carbopol^{®} contenant de l'acétate de prednisolone
   D'autres polymères peuvent être utilisés dans le but de retarder la libération du principe actif tels que l'acide alginique (Cartéol^{®} LP), et les gommes de gellane (Timoptol^{®}).
   Dans certains cas, il aurait été mis en évidence une certaine bio-adhésion des matériaux utilisés, au niveau de la cornée. Ce serait le cas du Carbopol^{®} (HO-WAH HUI et Coll. [7]). Mais le mécanisme de bio-adhésion ne serait pas parfaitement établi.
- des émulsions ou pseudo-latex. Ce sont des émulsions contenant des micro-billes à base d'acétophtalate de cellulose ou autres tels que des dérivés polymétacryliques, sur lesquelles est fixé le principe actif. Ces particules en suspension se déposent dans le cul-de-sac conjonctival et libèrent progressivement le principe actif.
- Des inserts tels que :
   - des matrices hydrophiles pré-trempées ou lentilles hydrophiles qui sont considérées comme des réservoirs car elles sont capables de s'hydrater à près de 85%. Cependant le relarguage d'un principe actif contenu dans ce réservoir est rapide
   - des implants solubles érodables imprégnés de principes actifs (plaques ovales ou bâtonnets). Placés dans le cul-de-sac conjonctival, ils s'imbibent rapidement de liquide lacrymal. La libération du principe actif se fait par dissolution progressive du support.

Un exemple concret de ce système est la spécialité Lacrisert^{®} à base d'hydroxypropylcellulose.

Dans le cas présent, les émulsions ou pseudo-latex ainsi que les inserts, ne sont pas définis comme des systèmes bio-adhésifs à libération prolongée. Leur activité prolongée provient du fait qu'ils sont maintenus en place au niveau de l'oeil dans le cul-de-sac conjonctival qui joue dans ce cas le rôle de réservoir de principes actifs. A ce niveau aucune combinaison physique ou chimique est mise en oeuvre avec l'un des composants biologiques de l'oeil.

Concernant la muqueuse nasale peu de choses ont été explorées en raison des effets secondaires que peut entraîner l'application de substances qui, généralement dans le cas des formes à action prolongées, sont des produits :
- insolubles en milieu aqueux telles que les celluloses ;
- présentant des viscosités non compatibles avec celles de l'organisme.

Ainsi un risque de passage de ces substances dans les voies respiratoires est à craindre pouvant entraîner une obstruction des bronches et bronchioles.

Cependant des études ont été menées avec le Carbopol^{®} (NAGAI et Coll. [8] et CHU et Coll. [9])). La biodisponibilité des principes actifs serait augmentée lors d'une administration à l'état :
- de poudres : mélange principe actif/Carbopol^{®}
- et d'hydrogels.

Le brevet Américain US 4,226,848 fait mention de l'utilisation de dérivés cellulosiques et de polymères acryliques pour une application nasale bio-adhésive.

Contrairement à la voie oculaire ou à la voie nasale où dans la majorité des cas les préparations bio-adhésives sont présentées sous forme de gels ou de crèmes, les formes dites bio-adhésives pour la voie buccale seraient en majorité présentées sous forme de comprimés ou de patches. Les ingrédients dits bio-adhésifs testés dans ce cadre d'application sont :
- le Carbopol^{®}
- l'hydroxypropylmethylcellulose
- le chitosan
- et la gomme d'acacia.

Quelques formes pâteuses existent dont les principaux excipients bio-adhésifs utilisés sont :
- les dérivés cellulosiques entre autre, la carboxyméthylcellulose
- des glycérides de très grande pureté qui ont la propriété de gélifier au contact de la salive
- et le Carbopol^{®}

Cependant même si les comprimés et les patches assurent une libération prolongée du principe actif dans la cavité buccale, le maintien pendant 8 heures d'une telle forme dans la bouche n'est pas concevable pour un patient. D'autre part les formes pâteuses ne résistent pas à l'action de « lessivage » de la muqueuse par la salive et n'est donc pas maintenues in situ sur une période de 8 heures.

Dans le cas de la voie vaginale les formes pouvant être mises en oeuvre sont identiques à celles de la voie buccale :
- comprimés
- formes pâteuses

Comme précédemment les comprimés peuvent très bien présentés une libération prolongée *in situ*.

Cependant l'administration d'une telle forme n'est pas pratique pour la patiente et un rejet peu avoir lieu au bout d'un certain temps.

Dans le cas des formes pâteuses nous retrouvons les mêmes excipients bio-adhésifs que pour la voie buccale ou oculaire. Cependant comme pour la voie buccale, les sécrétions vaginales ne permettent pas le maintien en place de telles préparations pour une longue durée d'action.

En dehors des suppositoires, les formes appliquées localement au niveau rectal sont généralement des crèmes ou des gels pour le traitement des hémorroïdes. Cette voie peut être aussi utilisée dans le but d'une action systémique de principes actifs qui, administrés par voie orale, sont rapidement dégradés au niveau hépatique. La voie rectale évite ce problème.

A ce jour très peu de formes à libération prolongée ont été développées pour cette voie. Des travaux ont été réalisés par HOSNY et coll.(10) en utilisant comme agent bio-adhésif, les Carbopol^{®} pour l'administration par voie rectale de l'indométhacine.

Ainsi après une étude approfondie de la littérature dans le domaine des formes à libération prolongée, il n'a pas été permis de mettre en évidence une solution efficace pour l'obtention d'une forme à libération prolongée de longue durée, supérieure à 2 heures, en raison du phénomène de lessivage des muqueuses par les sécrétions locales.

Partant de cet état de fait et pour y remédier, l'invention propose un concept original de compositions liquides visqueuses destinées à la réalisation de formes pâteuses à action et/ou à libération prolongée pour applications locales. Ces compositions sont remarquables en ce que l'action durable et/ou la libération prolongée du principe actif est obtenue par la formation *in situ* d'un film matriciel à pouvoir bio-adhésif renforcé, plus ou moins visqueux et biodégradable. Ledit caractère bio-adhésif renforcé est obtenu par une réaction de complexation de l'agent matriciel avec un des composants des sécrétions locales ou de la muqueuse, conduisant sous l'effet d'un «lessivage» permanent des muqueuses par les sécrétions, à une action et/ou une libération sur une période supérieure à 2 heures du principe actif préalablement dissous ou dispersé à l'aide de solvant, cette action et/ou cette libération étant modulable par incorporation d'adjuvants appropriés.

Ainsi la présente invention a pour but de réaliser *in situ*, après application de la préparation, un film matriciel à pouvoir bio-adhésif renforcé et biodégradable, dont l'action et/ou la libération du principe actif est, dans la mesure du possible, indépendante du pH et/ou indépendante de l'action des sécrétions des muqueuses, en fonction des excipients utilisés pour renforcer la solidité dudit film matriciel bio-adhésif.

La bio-adhésion renforcée est telle que celle-ci est quasi instantanée dès application sur les muqueuses.

On entend par «bio-adhésion» l'habilité qu'a une substance biologique ou synthétique à «coller» à une membrane biologique ou muqueuse.

On entend par «renforcé» la création de liaisons supplémentaires avec le support représenté par la muqueuse, autres que celles observées avec les excipients bio-adhésifs classiques.

On entend par «film matriciel», la formation d'un réseau tridimensionnel plus ou moins solide, plus ou moins épais, et plus ou moins poreux, dans lequel est inclus la substance active.

On entend par «biodégradable» la dégradation d'un support engendrée par un mécanisme biologique tel que l'action des enzymes mais aussi par un mécanisme d'érosion mécanique dû au «lessivage» des sécrétions de l'organisme.

On entend par «lessivage» un passage répété d'une solution sur un même support jusqu'à épuisement total de ce dernier.

Cette invention est applicable aussi bien aux préparations destinées à la muqueuse buccale qu'à la muqueuse nasale, vaginale, rectale et à la cornée.

Cette invention est basée sur le fait que certaines substances à l'état solide ou à l'état liquide ont la propriété de se complexer avec certaines molécules des muqueuses lorsqu'elles sont appliquées sur ces dernières. Ainsi il y a fixation de l'agent matriciel à la surface des muqueuses formant ainsi un réseau tridimensionnel dont l'action et/ou à partir duquel le principe actif diffuse progressivement dans le temps. Les substances mises en oeuvre sont en majorité des matières d'origine naturelle très utilisées dans le domaine pharmaceutique, cosmétique et diététique.

On entend par «complexer» la formation d'une liaison chimique autre que les liaisons hydrogènes rencontrées avec la plupart des autres agents dits bio-adhésifs. Cette liaison est caractérisée en ce que son énergie est intermédiaire entre l'énergie d'une liaison de covalence et l'énergie d'une liaison hydrogène conduisant ainsi à une structure plus résistante au phénomène de «lessivage».

Ainsi, l'invention a pour objet une composition liquide visqueuse à pouvoir bio-adhésif renforcé, pour une application locale sous forme pâteuse à libération prolongée d'un principe actif, caractérisée en ce qu'elle comprend au moins un agent matriciel, un milieu d'hydratation de l'agent matriciel, au moins un principe actif et au moins un tensioactif de la famille des phospholipides permettant de renforcer les propriétés bioadhésives des carraghénanes.

L'obtention desdits films matriciels bio-adhésifs à libération prolongée faisant l'objet de la présente invention font appel à des substances dites agents matriciels, qui au contact des muqueuses présentent un pouvoir bio-adhésif renforcé.

Ces substances peuvent être utilisées seules et donnent naissance à un film visqueux ou à une structure plus ou moins solide, dans laquelle le ou les principes actifs sont dissous ou dispersés.

Ces mêmes substances peuvent être utilisées en association avec d'autres excipients ayant pour but de renforcer la structure dudit film matriciel et leur propriété bio-adhésive.

En association avec d'autres excipients, ces agents matricielles jouent le rôle de «liant».

On entend par «liant» des substances agissant comme des ciments entre les particules d'un réseau dans le but de renforcer une structure plus ou moins solide.

Ainsi ces substances matricielles évitent la dispersion des autres excipients au sein des sécrétions de l'organisme par leur emprisonnement dans la gangue visqueuse ou la structure spongieuse formée à la surface de la muqueuse.

De ce fait, en fonction de la solidité du film matriciel obtenu, l'action dudit film ou la libération d'un principe actif inclus dans un tel système peut varier entre 1 à 48 heures en fonction du site d'action de ladite préparation.

Préférentiellement selon l'invention, le temps de libération du principe actif est compris entre 2 et 12 heures pour les voies buccales, nasale et oculaire et le temps de libération du principe actif est supérieur à 12 heures pour la voie vaginale.

Les agents matriciels permettant d'obtenir lesdits films et jouant le rôle de «liant» appartiennent à la classe des polymères naturels, les polysaccharides:

Les polysaccharides retenus dans le cadre de cette invention sont les carraghénanes.

Les carraghénanes sont connus depuis plus de 600 ans dans le domaine médical et en alimentation en particulier pour leur propriété originale qui consistait à gélifier le lait par simple chauffage.

Ce sont des polysaccharides, polymères du galactose plus ou moins sulfatés.

Les carraghénanes sont extraits à partir de différentes algues : *Chondrus crispus, Gigartina stellata, Gigartina acicularis, Gigartina skottsbergii, Gigartina pistillata, Gigartina chamissoi, Iridea, Eucheuma cottoni, Eucheuma spinosum.*

Le procédé d'extraction mis en oeuvre conduit à différents types de carraghénanes dont le squelette de base est une chaîne de résidus D-galactose liés alternativement en α-(1-3) et β-(1-4).

Les différentes qualités sont dues à la quantité et à la position des groupements sulfates et à la présence ou non d'un pont 3,6 anhydro sur le galactose lié en 1 et 4.

La proportion des différents groupes sulfates et du pont 3,6 anhydrogalactose a permis d'isoler différents types de carraghénanes. Ce sont les iota-, kappa-, lambda-, bêta-, nu- et mu-carraghénanes.

Les formes lambda- présentent beaucoup de groupements soufrés comparées aux formes kappa-. Les formes iota- sont intermédiaires.

Les formes mu- et nu- sont en plus faibles quantités et sont considérées comme impuretés diminuant l'effet gélifiant des formes iota- et kappa-.

Les types de carraghénanes retenus pour la présente invention sont les lambda- et les iota-carraghénanes.

Comparés aux kappa-carraghénanes, les lambda- et les iota-carraghénanes ne présentent aucun phénomène de synérèse.

Les lambda-carraghénanes ne présente pas de propriétés gélifiantes mais épaississantes.

Dans le cas des iota-carraghénanes, la propriété gélifiante ne se développe que si la préparation est soumise à la chaleur.

Que ce soit les iota- ou les lambda-carraghénanes, ce sont des substances hydrocolloïdes avides d'eau.

Par conséquent au contact des muqueuses, ces substances vont avoir la possibilité de développer des propriétés bio-adhésives comme les excipients bio-adhésifs classiques tels que le Carbopol^{®} ou la carboxyméthylcellulose définis, eux aussi, comme des polymères susceptibles de former des réseaux tridimensionnels plus ou moins solides.

Le mécanisme classique de bio-adhésion des excipients dits bio-adhésifs est défini comme étant une interaction dudit excipient avec le mucus recouvrant les muqueuses de l'organisme. Ce mucus est généralement hautement hydraté et présente une certaine viscosité due à la présence d'une glycoprotéine, la mucine.

De part leur nature chimique, polymères de haut poids moléculaire, ces excipients dits bio-adhésifs sont avides d'eau. Ainsi au contact des muqueuses, ces polymères gonflent rapidement avec création de liaisons hydrogènes entre les groupements hydrophiles du polymère et ceux du mucus et entre autres ceux de la mucine. De ce fait il y a formation d'un réseau tridimensionnel provenant d'une interaction polymère/mucine.

Cependant les liaisons hydrogènes sont des liaisons de faible énergie. Par conséquent une dilution du milieu ou un «lessivage» constant d'un support va conduire à une rupture rapide de ces liaisons diminuant ainsi le caractère bio-adhésif de ces excipients.

Par contre dans le cas des iota- et lambda-carraghénanes le caractère bio-adhésif est dit «renforcé» car hormis la création des liaisons hydrogènes observée avec les excipients classiques, d'autres liaisons se forment avec le support.

En effet, il a été mentionné que les iota- et lambda-carraghénanes possédaient des groupements sulfates sur le squelette de la molécule. Ces groupements chimiques sont très réactifs et donnent naissance à des réactions de complexation avec certaines molécules présentant des protons libres sur certains de leurs atomes tel que l'azote et le soufre. Du fait de la présence de ces protons libres, N²⁺ ou S⁴⁺, des groupements anioniques tels que les groupements sulfates des carraghénanes, SO₄²⁻, réagissent très fortement avec ces molécules.

Ainsi les iota- et lambda-carraghénanes en contact avec la mucine et de la muqueuse, porteuses dans les deux cas d'atomes d'azote, vont donner naissance à un réseau tridimensionnel constitué :
- de liaisons hydrogènes créées entre les groupements OH du polysaccharide et ceux de la mucine et de l'eau présente dans le mucus
- de liaisons de complexation entre les groupements sulfates du polysaccharide et les atomes d'azote de la mucine et de la muqueuse.

Le caractère bio-adhésif «renforcé» a été mis en évidence par une étude comparative entre le Carbopol^{®} 934P NF et les lambda-carraghénanes (Benvisco^{®} LPB 2301).

L'étude repose sur la réalisation de 2 solutions ayant une viscosité définie de 3000 mPa.

La viscosité de la solution n'a pas été choisie arbitrairement mais en fonction des propriétés mécaniques finales de la solution : solution facilement diffusible (sprayable).

Ainsi pour une viscosité de 3 Pa·s (3000 cPs), les concentrations en agents bio-adhésifs sont :
- 2,0% pour le lambda-carraghénanes.
- 0,6% pour le Carbopol^{®}

Au-delà de ces valeurs, les produits obtenus ne présentent plus l'aspect de solutions visqueuses mais de gels.

Contrairement à toutes les études réalisées sur la bio-adhésivité, sur support inox, l'étude de la bio-adhésivité de ces solutions a été réalisée sur membrane biologique de cellulose (membrane osmotique) imprégnée d'une solution de mucine à 5% dans un tampon phosphate pH 6.8.

L'imprégnation de la membrane est effectuée dans les 30 secondes avant le dépôt des solutions.

L'imprégnation de la membrane est telle qu'un film liquide se forme à la surface de cette dernière simulant ainsi ce qui se passe au niveau des différentes muqueuses : aspect humide des muqueuses.

0,5 ml de solution à tester sont déposés à la surface de cette membrane imprégnée, à 6 cm du bord inférieur.

Le caractère «renforcée» de la bio-adhésivité est testé par un «lessivage» du dépôt par un milieu tamponné de pH 6,8 simulant les différentes sécrétions de l'organisme.

Le «lessivage» de ces dépôts est réalisé en utilisant l'appareil de désagrégation des comprimés décrit à la Pharmacopée Européenne, 4^{ème} édition.

Cet appareil décrit un mouvement de va-et-vient de haut en bas à raison de 30 par minutes et d'une amplitude de 12 cm.

La membrane imprégnée est fixée sur un support rigide (plaque de verre), lui-même fixé à la verticale sur le levier décrivant le mouvement de va-et-vient.

L'appareil de désagrégation ainsi qu'un chronomètre, sont déclenchés lorsque le surplus de dépôt commence à s'égoutter à la partie inférieure de la membrane.

Hormis l'étude comparative fait entre le Carbopol^{®} et les lambda-carraghénanes sur membrane imprégnée, une étude parallèle a été réalisée sur une membrane simplement humidifiée.

On entend par «simplement humidifiée» l'absence de film aqueux à la surface de la membrane.

Contre toute attente, les lambda-carraghénanes au contact du film liquide à la surface de la membrane biologique présentent un temps de contact nettement plus important que le Carbopol^{®} mis dans les mêmes conditions.

Une différence significative de 2 minutes 36 secondes (2'36") est observée dans le cas du Carbopol^{®}, entre les temps de contact sur membrane simplement humide et sur membrane imprégnée contre une différence non significative de 1 minute 45 secondes (1'45") pour les lambda-carraghénanes.

| | Membrane humide | Membrane imprégnée |
|---|---|---|
| Lambda-carraghénanes | 9'43" ± 0'47" | 8'52" ± 0'16" |
| Carbopol^{®} | 9'29" ± 0'43" | 6'52" ± 0'9" |

Cette différence de comportement est d'autant plus significative que les carraghénanes ont un comportement rhéologique tout à fait différent de celui du Carbopol^{®}

En effet, les préparations liquides à base de carraghénanes sont caractérisés comme étant des produits newtoniens c'est-à-dire qu'ils s'écoulent librement sous l'effet de leur seul masse ce qui n'est pas le cas du Carbopol.

D'autre part ces mêmes produits sont aussi caractérisées comme étant des solutions thixotropes à savoir que sous l'effet d'une agitation, ces dites solutions ayant l'aspect d'un solide au repos, se liquéfient rapidement. Cette propriété n'est pas observée avec le Carbopol.

Ainsi après dépôt de la solution de carraghénanes à la surface de la membrane humide, cet écoulement a clairement été observé dès mise en position verticale du support et s'est poursuivi dès le déclenchement de l'appareil. Par contre dans le cas du Carbopol^{®}, cet écoulement était nettement moins important après déclenchement de l'appareil.

Or, contre toute attente, sur membrane imprégnée, le film liquide mobile à la surface de la membrane, libre de tout écoulement en position verticale, a vu son écoulement entravé par la présence des carraghénanes à sa surface. Contrairement aux résultats observés sur la membrane humide, l'écoulement du dépôt est pratiquement inexistant lorsque l'appareil est déclenché.

Dans le cas du Carbopol^{®}, un résultat inverse est observé. Le film liquide à la surface de la membrane n'est pas entravé dans son écoulement entraînant ainsi avec lui le dépôt effectué.

Cette propriété de bio-adhésivité a été aussi démontrée par l'enregistrement des temps d'écoulement d'une solution à base de carraghénanes sur un plan incliné à 45° recouverte de la même membrane biologique imprégnée ou non de mucine. Des différences de temps significatives ont été notées entre l'écoulement sur membrane non imprégnée et sur membrane imprégnée.

| **Concentration en carraghénanes** | **Sans mucine** | **Avec mucine** | **Aptitude à l'adhésion Fₐₐ** | | **Facteur de bio-adhésivité F_{ba}** | |
|---|---|---|---|---|---|---|
| | | | **Données** | **Moyenne** | **Données** | **Moyennes** |
| | 38" | 54" | 16" | 12" | 1.42 | 1.31 |
| 2% | 43" | 52" | 9" | ±3" | 1.21 | ±0.11 |
| | 41" | 53"' | 12" | 28.47% | 1.29 | 8.11 % |
| | 5'52 | 7'58 | 126" | 122" | 1.36 | 1.34 |
| 3% | 5'59 | 8'01 | 122" | ±3" | 1.34 | ±0.02 |
| | 6'03 | 8'02 | 119" | 2.87% | 1.33 | 1.13% |
| | 17'58 | 22'05 | 247" | 251" | 1.23 | 1.23 |
| 5% | 17'59 | 22'14 | 255" | ±4" | 1.24 | ±0.005 |
| | 18'08 | 22'19 | 251" | 1.59% | 1.23 | 0.47% |

Si les carraghénanes font l'objet d'un certain nombre de brevets dans des domaines les plus diverses autres que la pharmacie, la cosmétique et la diététique, ces derniers sont cependant peu utilisé dans la réalisation des formes à libération prolongée et encore moins pour la réalisation de système bio-adhésif à libération prolongée.

En effet, dans le domaine des formes à libération prolongée nous pouvons citer entre autres, la demande internationale WO 03101424 faisant mention des carraghénanes pour la réalisation de matrices à libération prolongée se présentant sous forme solide.

Dans le cas du brevet US 6,355,272, les carraghénanes sont complexés avec le principe actif qui est progressivement libéré dans le temps au niveau du tube digestif.

Dans le même ordre d'idée la demande internationale WO 0100177 protège l'association amoxicycline/carraghénanes en vue d'une libération prolongée de l'antibiotique.

La demande FR2542616 décrit une composition pour l'application locale aux muqueuses, en particulier aux muqueuses orales et génitales, comprenant un antibiotique et un carraghénane.

Hercules dans son brevet US 6,358,525 protège différentes compositions à base d'hydroxypropylcellulose et d'un hydrocolloïde tels que les carraghénanes, dans le but de ralentir la mise à disposition de l'organisme, des substances actives.

La demande de brevet US2004019010 utilise les gels de carraghénanes comme substitut de l'humeur vitrée de l'oeil lors de chirurgies oculaires, telle que la cataracte. L'humeur vitrée est substituée par ces gels qui, progressivement dans le temps, voient leur viscosité diminuée pour éviter une trop forte pression intraoculaire. A ces gels peuvent être associés des substances actives telles que des anti-inflammatoires, des antibiotiques, etc., qui évitent ainsi toutes complications consécutives à l'opération.

Le brevet US 5,403,841 protège les carraghénanes pour l'application ophtalmique de certains principes actifs. Dans le cas présent, il n'est pas revendiqué une bio-adhésion des carraghénanes mais une augmentation quasi instantanée de la viscosité de la solution à base de carraghénanes instillée dans l'oeil.

De même le brevet EP424043 fait bien mention de l'utilisation des carraghénanes pour augmenter le temps de libération des principes au niveau de l'oeil. Ce résultat serait dû à une augmentation de la viscosité du liquide lacrymale par interaction des carraghénanes avec les protéines des larmes, entre autre les lysozymes. En aucun cas le processus d'interaction n'est décrit. En effet il n'est nullement fait mention :
- de création de liaisons hydrogènes
- de réaction de complexation
- de réaction de précipitation qui peut être aussi un mécanisme d'augmentation de la biodisponibilité par formation de microcristaux in situ.

D'autre part il n'est absolument pas fait mention d'une activité bio-adhésive de ces préparations au niveau de la cornée dont la couche extérieure est recouverte d'un mélange de lipides et de mucine.

Au niveau buccal, il est fait mention dans le brevet US 5,672,356 de l'utilisation des carraghénanes en tant qu'agent gélifiant retardant la libération du principe actif, la bio-adhésion étant apportée par le copolymère d'éther méthylvinylique et d'anhydre maléique.

Au niveau de l'oesophage le brevet US 6,610,667 protège une composition dont le principal agent de bio-adhésion est l'alginate et a un degré moindre d'autres hydrocolloïdes tels que la gomme de xanthan, les galactomananes, les glucomananes et les carraghénanes. L'application est principalement centrée autour de l'association alginate/gomme xanthane ou alginate/galactomananes ou glucomananes. D'autre part l'oesophage est loin d'être une cavité d'accès facile de l'extérieur.

La demande internationale W02004/075920 utilise les carraghénanes comme vecteur de principes actifs au niveau pulmonaire dans le but de retarder leur libération. Là aussi, les poumons sont loin d'être considérés comme étant une cavité au même titre que la cavité buccale, vaginale, rectale, donc loin d'être d'accès facile de l'extérieur. D'autre part les sécrétions pulmonaires sont nettement moins importantes que celles observées à partir des glandes salivaires ou lacrymales.

D'autres brevets tel que le brevet EP1452168 ont pour objet l'applications cutanées des carraghénanes. La demande de brevet US2002071861 met en oeuvre les carraghénanes mais l'aspect bio-adhésif de ces préparations est apporté par la carboxyméthylcellulose, l'hydroxypropylméthylcellulose et le Carbopol^{®}.

Enfin les brevets US 6,159,491, US 5,069,906 et US 4,983,393 font mention de l'utilisation des carraghénanes dans des systèmes à libération prolongée destinés à la voie vaginale.

Le Brevet US 6,159,491 met en oeuvre une combinaison du Carbopol^{®} (Polycarbophil^{®}) avec des carraghénanes et de l'agarose de haute pureté dans le but d'avoir une libération en deux temps. Dans le cas présent les carraghénanes sont utilisés comme gélifiants retardant la libération des substances actives et le Carbopol^{®} comme bio-adhésif.

Les Brevets US 5,069,906 et US 4,983,393 protègent les carraghénanes uniquement en tant qu'agent matriciel retardant la libération des actifs. Le caractère bio-adhésif n'est pas mentionné.

Dans la présente invention, en fonction de la zone traitée, la concentration en agent matriciel, particulièrement en carraghénanes, dans le milieu varie de 0,5% à 30% par rapport à la masse finale de la préparation.

Le solvant d'hydratation de l'agent matriciel, particulièrement des carraghénanes, peut être aqueux ou hydro-alcoolique. La proportion de la phase alcoolique peut varier de 10% à 90% en masse par rapport au volume totale de la phase hydratante.

La phase alcoolique peut être représentée par l'alcool éthylique et l'alcool isopropylique.

L'addition de certains ions peut permettre une meilleure hydratation des carraghénanes et en même temps permet d'augmenter leur concentration dans le milieu.

Les agents favorisant cette hydratation appartiennent à la classe des alcalins et des alcalino-terreux. Ce sont entre autres :
- les sels sodiques et potassiques des acides chlorhydrique, sulfurique, nitrique, phosphorique, citrique et dérivés;
- et les d'hydroxydes de sodium et de potassium.

La proportion d'ions alcalins et alcalino-terreux pouvant être introduite dans le milieu varie entre 0% à 50% en masse par rapport à la masse totale de la préparation.

La phase aqueuse utilisée peut être tamponnée pour favoriser la stabilité des substances actives mais aussi la stabilité de l'agent matriciel.

En effet, les carraghénanes en présence de dextrose en milieu neutre, subissent une hydrolyse progressive dans le temps, accrue par l'action de la chaleur.

C'est ainsi qu'en milieu neutre et sur une période de 24 heures on observe une diminution de la viscosité du produit par une hydrolyse progressive des carraghénanes libérant dans le milieu des radicaux acides.

Dans le cadre de solutions tampons acide, les compositions suivantes peuvent être :
- tampon acide chlorhydrique/chlorure de sodium ou acide chlorhydrique/phtalate de potassium ou acide chlorhydrique/glycocolle.
- tampon acide citrique/citrate ou acide citrique/hydroxyde de sodium
- tampon acide lactique/lactate.

La proportion des différents composants permet de maintenir un pH acide compris entre 2 et 5.

Dans le cas des carraghénanes une meilleure stabilité est observée en milieu neutre ou basique.

Les solutions tampons qui peuvent être ainsi utilisées répondent aux compositions suivantes :
- tampon phosphates : phosphate de sodium ou de potassium
- tampon carbonates : bicarbonate/carbonate
- tampon phtalate : diphtalate de potassium/acide chlorhydrique
- tampon borates : acide borique/borate de sodium

La valeur du pH du milieu tamponné peut varier de 5 à 12.

La présente invention est destinée à l'administration d'un certain nombre de substances actives.

Les substances actives pouvant faire l'objet d'une telle mise en forme appartiennent à certaines catégories pharmacologiques à savoir les antalgiques, les anti-inflammatoires, les antispasmodiques, les cytotoxiques, les antibiotiques, les antifongiques, les antiseptiques, les antiparasitaires, les hormones, les anti-viraux, les migraineux, les antiallergiques, les analeptiques respiratoires, les spermicides, les antihémorroïdaires, les vasoconstricteurs, les vasodilatateurs, les antiprurigineux, les utérorelaxants, les antiglaucomateux, les mydriatiques, les antiasthmatiques.

Ces substances peuvent être incorporées à l'état dissous dans la phase aqueuse ou hydro-alcoolique de la préparation faisant l'objet de la présente invention ou à l'état solide dispersé dans le film matriciel.

Si un certain nombre de ces substances peut être solubilisé dans le milieu d'hydratation des carraghénanes, d'autres nécessitent une solubilisation dans une phase organique.

Parmi les solvants organiques pouvant être mis en oeuvre sans danger pour l'organisme humain sont retenus :
- les huiles végétales, huiles végétales hydrogénées, huiles végétales éthoxylées : huile d'olive, de noisette, de noix de coco, huile de ricin, huile de soja, huile de sésame, etc.
- les huiles minérales : paraffine, isoparaffine, cycloparaffine, huiles de silicones, isohexadécane, isododécane, et dérivés, etc.
- les huiles naturelles, squalane, hexaméthyltétracosane, les mono-, di- et triglycérides, etc.
- huiles synthétiques : polyisobutène, polyisobuténe hydrogénée, etc.
- et autres solvants : éthanol, propanol-1, propanol-2, polypropylène, propylène carbonate, diméthyl isosorbide éther, polyoxyéthylène glycols (Macrogols), glycérol, esters d'acide gras du polyéthylène, esters d'acide gras du propylène glycol, dicaprylate/dicaprate de propylène glycol, caprylate/caprate de glycérol, esters d'acide gras du polyoxyéthylène/polyoxypropylène glycol, triacétin, myristate d'isopropyle, glycofurol, esters liquide d'acide gras, acétate d'éthyle, butanol, propylène glycol acétate, butyl acétate, éthylèneglycol monobutyl éther, éthyle lactate, butyl acétate, éther monoéthylique du diéthylèneglycol, glycérine mono oléate, glycérine linoléate, esters d'acide gras et de glycérol, esters d'acide gras de glycérol et de PEG etc.

La proportion de ces différents solvants utilisés dans ces préparations, dépend de la solubilité des principes actifs et peut varier de 1% à 50% en volume par rapport au volume total de la phase hydratante.

Dans certains cas, ces solvants nécessitent l'utilisation de tensioactifs pour éviter tout déphasage entre la phase hydratante et la solution organique d'actifs.

Les tensioactifs pouvant être utilisés dans la présente invention sont :
- les tensioactifs non ioniques :
   - des esters de sorbitane : polysorbates, spans, tweens, etc...
   - des acides gras polyéthoxylés : stéarate de PEG 8 au stéarate de PEG 100;
   - des alcools gras polyéthoxylés : mélange d'éther de monolaurate de PEG ayant de 4 à 23 groupes oxyéthylènés sur la chaîne polyoxyéthylénique, etc...
   - des esters de glycol : stéarate de méthylglycol;
   - des esters de glycérol : monostéarate de glycérol, stéarate de PEG 75, stéarate de glycol et de PEG 6-32, etc...
   - des esters de PEG;
   - des esters de saccharose;
   - des éthers d'alcool gras et de PEG : Brij;
   - des éthers d'alkyl phénol et de PEG;
   - des tensioactifs présentant une fonction amide :
      ∘ monoéthanolamide d'acide gras de coprah, d'acide laurique, etc...
      ∘ diéthanolamide d'acide myristique, d'acide laurique, etc...
      ∘ mono-isopropanolamine d'acide laurique.
   - les phospholipides tels la phosphatidylcholine, la phosphatidylsérine,
- les tensioactifs ioniques :
   - des dérivés sulfatés : le laurylsulfate de sodium et ses dérivés;
   - des dérivés sulfonés : dodécylsulfosuccinate de sodium et ses dérivés;
   - des ammoniums quaternaires : chlorure de cétyltriméthylammonium, laurylpyridinium, distéaryldiméthylammonium, etc...
- amphotères : bétaïne d'ammonium d'alkyldiméthyle de coprah, dérivés d'amides d'acide gras à structure bétaïnique, acide lauryl-α-iminodipropionique et ses dérivés, acide lauryl-myristyl-α-aminopropionique et ses dérivés, etc...

D'autre part les tensioactifs sont utilisés pour renforcer les propriétés bio-adhésives des carraghénanes, de la famille des phospholipides tel que la phosphatidylcholine.

Basé sur les mêmes tests que précédemment la phosphatidylcholine augmente le pouvoir bio-adhésif des carraghénanes de façon significative. Pour une même viscosité et une concentration moindre la différence de temps entre l'écoulement sur membrane imprégnée et sur membrane non imprégnée est de l'ordre de 414 secondes. En l'absence de phospholipides cette différence n'est que de 122 secondes

| **Viscosité** | **Concentration en carraghénane** | **Sans mucine** | **Avec mucine** | **Aptitude à l'adhésion Fₐₐ** | | **Facteur de bioadhésivité F_{ba}** | |
|---|---|---|---|---|---|---|---|
| | | | | **Données** | **Moyenne** | **Données** | **Moyennes** |
| | **2%+** | 9'38 | 17'11 | 453" | 414" | 1.78 | 1.72 |
| **2533 cPs** | phospholipides | 9'54 | 15'31 | 337" | ±67" | 1.56 | ±0.14 |
| | | 9'12 | 16'46 | 454" | 16.22% | 1.82 | 8.13% |
| | | 5'52 | 7'58 | 126" | 122" | 1.36 | 1.34 |
| **2625 cPs** | **3%** | 5'59 | 8'01 | 122" | ±3" | 1.34 | ±0.02 |
| | | 6'03 | 8'02 | 119" | 287% | 1.33 | 1.13% |

La quantité de ces substances utilisée pour favoriser la solubilisation ou la dispersion des principes actifs ainsi que pour accroître le pouvoir bio-adhésif des carraghénanes, peut varier de 0 à 50% en poids par rapport à la masse totale des excipients.

Hormis le fait que les principes actifs peuvent être solubilisés dans la phase hydratante ou dans un autre solvant, ceux-ci peuvent être aussi incorporés à l'état solide donnant naissance à une suspension bio-adhésive.

Par conséquent les principes actifs doivent répondre à un critère de granulométrie.

Ainsi la répartition granulométrique des poudres peut s'échelonner de 1 µm à 1000 µm avec une préférence comprise entre 1 µm et 250 µm.

Tel que cela a été mis en évidence lors d'un certain nombre d'essais, les carraghénanes donnent naissance à un film visqueux, d'aspect gélatineux et de consistance molle à consistance ferme en fonction de la concentration.

Un renforcement de cette structure peut être effectué par introduction dans le milieu de substances qui au contact des sécrétions vont accroître la solidité de ce réseau.

De nombreuses substances peuvent jouer ce rôle. Mais seuls les amidons ont été retenus en particulier leurs dérivés car ce sont des produits plus ou moins solubles dans le milieu d'hydratation qui renforcent la structure matricielle des carraghénanes grâce à leur aptitude à former des réseaux visqueux au contact de l'eau.

Ainsi les amidons natifs sont retenus comme agents de structuration dans ladite invention ainsi que leurs produits dérivés issus de :
- modifications physiques : pré-gélatinisation
- modifications chimiques :
   - réaction de dextrénisation chimiques ou enzymatiques
   - hydrolyse acide
   - réaction d'oxydation
   - réaction de substitution par :
      ∘ l'acide phosphorique
      ∘ l'acide adipique
      ∘ l'acide acétique
      ∘ des groupements hydroxypropyle ou hydroxyéthyle.

Ces différents agents de structuration peuvent être obtenus à partir des amidons de blé, de riz, de maïs, de manioc de pomme de terre.

Les quantités utilisées dans le but d'obtenir une action et/ou une libération du principe actif entre 2 et 48 heures peuvent varier de 0 à 50% en masse par rapport à la masse totale de la préparation

Un facteur important pour obtenir une structure compacte et rapide dans le temps, est la taille des particules de ces dites substances. En effet plus les particules sont fines, plus le pouvoir de gonflement est important et le réseau formé est beaucoup plus dense (moins d'interstices entre les particules).

Ainsi la taille des particules des amidons et amidons modifiés permettant d'obtenir un tel résultat doit être compris entre 1 µm et 1000 µm avec une préférence pour une taille comprise entre 1 µm et 100 µm.

Des adjuvants de conservation et des colorants peuvent être introduits dans la composition.

La proportion de conservateurs peut varier de 0% à 10% en masse par rapport à la masse totale de la préparation.

Les colorants peuvent être hydrosolubles ou fixés sur laque d'alumine ou autre support.

Le taux optimum de colorant requis se situe entre 0,01% et 5% en masse par rapport à la masse totale de la préparation.

Dans le cas des préparations destinées à la cavité buccale, des produits humectants peuvent être additionnés au milieu bio-adhésif.

On entend par « humectants » des substances qui apportent une certaine humidité au milieu dans lequel ils sont présents grâce à leur propriétés hygroscopiques intrinsèques à savoir, fixation de l'humidité de l'atmosphère environnante.

Ces produits ont la particularité de faciliter l'hydratation des carraghénanes ce qui se concrétise par une augmentation de la bio-adhésivité de ces derniers.

Ainsi basé sur les mêmes tests que précédemment, il apparaît que la glycérine, pour une même concentration en carraghénanes conduit à un temps d'écoulement sur la membrane imprégnée de mucine, nettement plus important qu'une simple préparation aqueuse de carraghénanes.

En effet sur une distance d'écoulement de 10 cm, la préparation n'atteint pas cette distance après 60 minutes de dépôt en raison d'une forte réaction avec le support

| **Concentration en carraghénanes** | **Sans mucine** | **Avec mucine** | **Aptitude à l'adhésion Fₐₐ** | | **Facteur de bio-adhésivité F_{ba}** | |
|---|---|---|---|---|---|---|
| | | | **Données** | **Moyenne** | **Données** | **Moyenne** |
| | 36'42 | 8.4 cm en 60' | / | | | |
| **3% + glycérine** | 36'35 | 8.7 cm en 60' | / | / | / | / |
| **15400 cPs** | 36'33 | 8.2 cm en 60' | / | | | |
| | 5'52 | 7'58 | 126" | 122" | 1.36 | 1.34 |
| **3%** | 5'59 | 8'01 | 122" | ±3" | 1.34 | ±0.02 |
| | 6'03 | 8'02 | 119" | 2.87% | 1.33 | 1.13% |

Sur une distance de 7.5 cm, il apparaît clairement que la différence d'écoulement entre les deux préparations est nettement significative. Le facteur de bio-adhésivité qui n'est autre que le rapport entre le temps d'écoulement avec mucine sur le temps d'écoulement sans mucine est nettement supérieur à celui des carraghénanes seuls que ce soit pour une même concentration en carraghénanes ou pour une même viscosité du milieu.

| **Concentration en carraghénanes** | **Sans mucine** | **Avec mucine** | **Aptitude à l'adhésion Fₐₐ** | | **Facteur de bio-adhésivité F_{ba}** | |
|---|---|---|---|---|---|---|
| | | | **Données** | **Moyenne** | **Données** | **Moyenne** |
| | 23'05 | 49'02 | 1557" | 1548" | 2.12 | 2.11 |
| **3% + glycérine** | 23'10 | 48'56 | 1546" | ±7" | 2.11 | ±0.01 |
| **15400 cPs** | 23'15 | 48'58 | 1543" | 0.47% | 2.10 | 0.47% |
| | 1'43 | 2'32 | 49" | 44" | 1.47 | 1.45 |
| **3%** | 1'38 | 2'24 | 46" | ±5" | 1.47 | ±0.04 |
| **2625 cPs** | 1'45 | 2'13 | 38" | 12.82% | 1.40 | 2.79% |
| | 10'01 | 13'49 | 228" | 219" | 1.38 | 1.38 |
| **5%** | 10'13 | 13'39 | 206" | ±11" | 1.37 | ±0.005 |
| **15933 cPs** | 9'44 | 13'28 | 224" | 5.34% | 1.38 | 0.42% |

Parmi ces substances se trouvent les polyols tel que la glycérine, le sorbitol, le maltitol, le xylitol, mannitol, etc..

Ces produits peuvent être utilisés à une concentration se situant entre 1 et 30% en masse par rapport à la masse totale de la phase liquide.

Ces préparations étant destinées entre autre à être appliquée sur la muqueuse buccale, des arômes ainsi que des édulcorants peuvent être additionnés au milieu bio-adhésif.

Les arômes peuvent être d'origine naturelle ou synthétique de même que les édulcorants.

Hormis le saccharose classiquement utilisé comme édulcorant, l'aspartam, l'acésulfam, le saccharinate de sodium et le cyclamate de sodium peuvent être retenu comme agents d'édulcoration.

En fonction de l'édulcorant utilisé, la concentration dans le milieu peut varier de 0,1% à 30% en masse par rapport à la masse totale de la préparation.

Les solutions ou suspensions ainsi réalisées donnant naissances *in situ* à des systèmes matriciels à libération prolongée, présentent des viscosités allant de 100 mPa et 500.000 mPa.

Ces solutions ou suspensions peuvent être conditionnées :
- en flacons multidosages : bouteilles ou tubes
- en dose unitaire : unidose, bottle-pack^{®}, tube canule à usage unique
- en spray : pulvérisation de liquide ou formation de mousse

De tels systèmes après application, conduissent à une action et/ou à la libération progressive du principe actif sur une période pouvant aller de 1 heure à 48 heures, cette cinétique de libération étant peu ou pas dépendante des facteurs biologiques environnants. Cette cinétique de dissolution peut être d'ordre zéro ou 1 selon le type d'excipients utilisés pour obtenir une telle libération.

Hormis l'utilisation de ces systèmes dans le cadre une libération prolongée d'un actif au sein d'une cavité, ces mêmes systèmes en l'absence de toute molécule thérapeutique, présentent un intérêt sur un plan mécanique et entre autres au niveau de la lubrification des muqueuses lorsque celles-ci sont atteintes d'une sécheresse, tel que la sécheresse buccale dans l'hyposialie, les sécheresses vaginales, les sécheresses nasales et les sécheresses cornéennes dans le cas de la maladie de Sjögren.

En effet de tels systèmes, et du fait du caractère intrinsèque lubrifiant des carraghénanes pouvant être amplifié par l'incorporation d'huiles minérales, végétales ou de tensioactifs, permettent de maintenir une action lubrifiante sur une période de 8 heures.

### Exemple de référence 1 : Gel hydratant bio-adhésif pour hyposialie

| | |
|---|---|
| Carraghénanes lambda | 5,00% |
| Arôme menthe | 0,02% |
| Arôme vanille | 0,50% |
| Aspartam | 0,01% |
| Eau déminéralisée | 94,47% |

### Exemple de référence 2 : Gel lubrifiant bio-adhésif pour hyposialie

| | |
|---|---|
| Carraghénanes lambda | 5,00% |
| Polysorbate 80 | 2,50% |
| Huile végétale | 2,50% |
| Arôme menthe | 0,02% |
| Arôme vanille | 0,50% |
| Aspartam | 0,01% |
| Eau déminéralisée | 89,47% |

### Exemple de référence n°3 : Gel hydratant bio-adhésif vaginale

| | |
|---|---|
| Carraghénanes lambda | 5,00% |
| Acide hyaluronique | 2,50% |
| Huile de paraffine | 1,00% |
| Monostéarate de glycérol auto-émulsionable | 8,00% |
| Parahydroxybenzoate de méthyle sodé | 0,08% |
| Parahydroxybenzoate de propyle sodé | 0,02% |
| Hydroxyde de sodium | QS pH 3,5 à 4,5 |
| Eau déminéralisée | 73,40% |

### Exemple de référence n°4 . Gel bio-adhésif pour mycoses buccales

| | |
|---|---|
| Carraghénanes lambda | 2,50% |
| Miconazole | 2,00% |
| Amidon prégélatinisé | 2,50% |
| Polysorbate 20 | 2,00% |
| Parahydroxybenzoate de méthyle sodé | 0,08% |
| Parahydroxybenzoate de propyle sodé | 0,02% |
| Ethanol à 96% V/V | 1,50% |
| Eau déminéralisée | 89,40% |

### BIBLIOGRAPHIE

(1) WANG E.S.N et Coll., Corneal absorption reinforcement of certain mydriatics, J. Pharm. Sci., 1970, 59, 11, p : 1559-1563
(2) HASS J.S., et Coll., The effect of methylcellulose on response to solutions of pilocarpine, S. Afr. Med. J., 1975, 49, p : 1259-1265
(3) GOLDBERG I. et Coll., efficacity and patient acceptance of pilocarpine gel, Am. J. Ophtalmol. 1979, 15, 3, p : 843-846
(4) MANDELL A.I.., et Coll., Muticlinic evaluation of pilocarpine gel, Invet. Ophtalmol. Vis. Sci., (Suppl.), avril 1979, p : 165
(5) MARCH W.F. et Coll., Duration of effect of pilocarpine gel, Arch. Ophtalmol., 1982, 100, 12, p : 1270-1271
(6) SCHOENWALD R.D. et Coll., Effect of particle size on ophtalmic bioavailability of dexamethasone suspension in rabbits, J. Pharm. Sci., 1980, 69, 4, p : 391-395
(7) HUI H.W., et Coll. Ocular Delivery of progesterone using a bioadhesive polymer, Intl. J. Pharma., 26 (1), 203-213, 1985
(8) NAGAI T., et Coll. Powder Dosage Form of insulin for nasal administration, J. Contr. Rel., 1(1), 15-22, 1984
(9) CHU J.S et Coll., viscometric study of polyacrylic acid systems and mucoadhesive sustained release gels, Pharmaceutical research, 9 (11), 1408-12, 1991
(10)HOSNY E.A, et Coll. Bioavailibility of sutained release indomethacin suppositories containing Polycarbophil®, Intl. J. Pharma., 113 (Jan 16), 209-213, 1995.

## Revendications

1. Composition liquide visqueuse à pouvoir bio-adhésif renforcé, pour une application locale sous forme pâteuse à libération prolongée d'un principe actif, **caractérisée en ce qu'**elle comprend :
(i) au moins un excipient bio-adhésif appartenant à la famille des carraghénanes permettant la formation *in situ* d'un film matriciel à pouvoir bio-adhésif renforcé par création de liaisons de complexation de l'excipient bio-adhésif et un des composants des sécrétions locales ou de la muqueuse,
(ii) un milieu d'hydratation de l'agent matriciel,
(iii) au moins un principe actif,
et (iiii) au moins un tensioactif de la famille des phospholipides permettant de renforcer les propriétés bio-adhésives des carraghénanes.

2. Composition liquide visqueuse selon la revendication 1, **caractérisée en ce que** les phospholipides sont choisis parmi les phosphatidylcholines.

3. Composition liquide visqueuse selon la revendication 2, **caractérisée en ce que** les phospholipides sont choisis parmi les phosphatidylsérines.

4. Composition liquide visqueuse selon la revendication 1, **caractérisée en ce que** le carraghénane est choisi parmi les lambda- et les iota-carraghénanes.

5. Composition liquide visqueuse selon la revendication 1, **caractérisée en ce que** le carraghénane est a une concentration comprise entre 0,5% et 30% en masse par rapport à la masse totale de la composition.

6. Composition liquide visqueuse selon l'une quelconque des revendications 1 à 5, **caractérisée en ce que** le milieu d'hydratation de l'agent matriciel est une solution aqueuse ou hydro-alcoolique.

7. Composition liquide visqueuse selon la revendication 6, **caractérisée en ce que** la phase hydro-alcoolique comprend de l'éthanol ou de l'alcool isopropylique.

8. Composition liquide visqueuse selon l'une quelconque des revendications 6 ou 7, **caractérisées en ce que** la proportion en phase alcoolique est comprise entre 10% et 90% en masse par rapport au volume total de la phase hydratante.

9. Composition liquide visqueuse selon l'une quelconque des revendications 1 à 8, **caractérisée en ce qu'**elle comprend en outre des ions alcalins ou alcalino-terreux, de préférence du sodium ou du potassium.

10. Composition liquide visqueuse selon la revendication 9, **caractérisée en ce que** la proportion d'ions alcalins ou alcalino-terreux varie de 0 à 50% en masse par rapport à la masse totale de la composition.

11. Composition liquide visqueuse selon l'une quelconque des revendications 9 ou 10, **caractérisée en ce que** l'ion alcalin ou alcalino-terreux est introduit sous forme d'hydroxyde ou d'un sel de l'acide chlorhydrique, sulfurique, nitrique, phosphorique, citrique et ou d'un dérivé.

12. Composition liquide visqueuse selon l'une quelconque des revendications 1 à 11, **caractérisée en ce que** la phase aqueuse du milieu d'hydratation est une solution tampon.

13. Composition liquide visqueuse selon la revendication 12, **caractérisée en ce que** la valeur du pH de la solution tampon varie de 2 à 12.

14. Composition liquide visqueuse selon selon l'une quelconque des revendications 12 ou 13, **caractérisée en ce que** la solution tampon est constituée par les couples acide chlorhydrique/chlorure de sodium, acide chlorhydrique/phtalate de potassium, acide chlorhydrique/glycocolle, acide citrique/citrates, acide citrique/hydroxyde de sodium, acide lactique/lactate, phosphate monosodique/phosphate disodique, phosphate monopotassique/phosphate dipotassique, bicarbonate/carbonate, diphtalate de potassium/acide ou acide borique/borate de sodium.

15. Composition liquide visqueuse selon l'une quelconque des revendications 1 à 14, **caractérisée en ce que** le principe actif est solubilisé dans la phase hydratante ou dans un solvant organique.

16. Composition liquide visqueuse selon la revendication 15, **caractérisée en ce que** le solvant organique est choisi parmi les huiles végétales, les huiles minérales, les huiles naturelles, les huiles synthétiques, les solvants classiques lipophiles, hydrophiles et hydro-lipophiles non toxiques.

17. Composition liquides visqueuse selon l'une quelconque des revendications 1 à 16, **caractérisée en ce qu'**elle comprend en outre au moins un deuxième tensioactif

18. Composition liquide visqueuse selon la revendication 17, **caractérisée en ce que** le deuxième tensioactif appartient à la classe des tensioactifs ioniques, non ioniques et amphotères.

19. Composition liquide visqueuse selon l'une quelconque des revendications 17 ou 18, **caractérisée en ce que** la concentration en tensioactifs est comprise entre 0 et 50% en masse par rapport à la masse totale des excipients.

20. Composition liquide visqueuse selon l'une quelconque des revendications 1 à 19, **caractérisée en ce que** le principe actif à l'état solide est dispersé sous forme de poudre d'une granulométrie comprise entre 1 µm et 1000 µm.

21. Composition liquide visqueuse selon la revendication 20, **caractérisée en ce que** la granulométrie du principe actif est comprise entre 1 µm et 250 µm.

22. Composition liquide visqueuse selon l'une quelconque des revendications 1 à 21, **caractérisée en ce qu'**elle comprend en outre de l'amidon de riz, de pomme de terre, de mais, de manioc, de blé et leurs dérivés.

23. Composition liquide visqueuse selon la revendication 22, **caractérisée en ce que** la concentration en amidon ou en amidon modifié est comprise entre 0 et 50% en masse par rapport à la masse totale de la composition.

24. Composition liquide visqueuse selon l'une quelconque des revendications 1 à 23, **caractérisée en ce qu'**elle comprend en au moins un agent de conservation, d'humectation, de coloration, d'aromatisation et/ou d'édulcoration.

25. Composition liquide visqueuse selon la revendication 24 **caractérisée en ce que** la concentration en conservateurs est comprise entre 0 et 10%.

26. Composition liquide visqueuse selon la revendication 24 **caractérisée en ce que** la concentration en agents humectants est comprise entre 1 et 30%.

27. Composition liquide visqueuse selon la revendication 24, **caractérisée en ce que** la concentration en colorants est comprise entre 0 et 5%.

28. Composition liquide visqueuse selon la revendication 24, **caractérisée en ce que** la concentration en édulcorants est comprise entre 0 et 30%.

29. Composition liquide visqueuse selon l'une quelconque des revendications 24 ou 28, **caractérisée en ce que** les édulcorants sont naturels ou synthétiques choisis parmi le saccharose, l'aspartam, l'acésulfam, le cyclamate de sodium ou le saccharinate de sodium.

30. Composition liquide visqueuse selon l'une quelconque des revendications 1 à 29 **caractérisée en ce qu'**elle présente une viscosité comprise entre 100 mPa et 500.000 mPa.

31. Composition liquide visqueuse selon l'une quelconque des revendications 1 à 30 **caractérisée en ce que** le temps de libération du principe actif est compris entre 2 et 12 heures pour les voies buccales, nasale et oculaire.

32. Composition liquide visqueuse selon l'une quelconque des revendications 1 à 31 **caractérisée en ce que** le temps de libération du principe actif est supérieur à 12 heures pour la voie vaginale.

33. Composition liquide visqueuse selon l'une quelconque des revendications 1 à 32, **caractérisée en ce que** le principe actif appartient à une classe thérapeutique choisie parmi les antalgiques, les anti-inflammatoires, les antispasmodiques, les cytotoxiques, les antibiotiques, les antifongiques, les antiseptiques, les antiparasitaires, les hormones, les anti-viraux, les migraineux, les antiallergiques, les analeptiques respiratoires, les spermicides, les antihémorroïdaires, les vasoconstricteurs, les vasodilatateurs, les antiprurigineux, les utérorelaxants, les antiglaucomateux, les mydriatiques, les antiasthmatiques.

## Claims

1. Viscous liquid composition having reinforced bio-adhesive capacity, for a local application in pasty form with prolonged release of an active ingredient, **characterized in that** it includes:
(i) at least one bio-adhesive excipient belonging to the family of carrageenans allowing the in situ formation of a matrix film with reinforced bio-adhesive capacity by creation of complexing bonds of the bio-adhesive excipient and one of the components of local secretions or of the mucous membrane,
(ii) a hydration medium of the matrix agent, (iii) at least one active ingredient,
and (iiii) at least one surface-active agent of the family of phospholipides allowing to reinforce the bio-adhesive properties of the carrageenans.

2. Viscous liquid composition according to claim 1, **characterized in that** the phospholipides are selected among the phosphatidylcholines.

3. Viscous liquid composition according to claim 2, **characterized in that** the phospholipides are selected among the phosphatidylserines.

4. Viscous liquid composition according to claim 1, **characterized in that** the carrageenan is selected among the lambda- and the iota-carrageenans.

5. Viscous liquid composition according to claim 1, **characterized in that** the carrageenan is at a concentration ranging between 0.5% and 30% in mass in relation to the total mass of the composition.

6. Viscous liquid composition according to any one of claims 1 to 5, **characterized in that** the hydration medium of the matrix agent is an aqueous solution or hydroalcoholic.

7. Viscous liquid composition according to claim 6, **characterized in that** the hydroalcoholic phase includes ethanol or isopropyl alcohol.

8. Viscous liquid composition according to any one of claims 6 or 7, **characterized in that** the proportion in alcoholic phase lies between 10% and 90% in mass in relation to the total volume of the hydrating phase.

9. Viscous liquid composition according to any one of claims 1 to 8, **characterized in that** it furthermore includes alkaline or alkaline-earth ions, preferably of sodium or potassium.

10. Viscous liquid composition according to claim 9, **characterized in that** the proportion of alkaline or alkaline-earth ions varies from 0 to 50% in mass in relation to the total mass of the composition.

11. Viscous liquid composition according to any one of claims 9 or 10, **characterized in that** the alkaline or alkaline-earth ion is introduced in the form of hydroxide or a salt of hydrochloric, sulphuric, nitric, phosphoric, citric acid and/or a derivative.

12. Viscous liquid composition according to any one of claims 1 to 11, **characterized in that** the aqueous phase of the hydration medium is a buffer solution.

13. Viscous liquid composition according to claim 12, **characterized in that** the value of the pH of the buffer solution varies from 2 to 12.

14. Viscous liquid composition according to any one of claims 12 or 13, **characterized in that** the buffer solution is constituted by the couples hydrochloric acid/sodium chloride, hydrochloric acid/potassium phthalate, hydrochloric acid/glycol, citric acid/citrates, citric acid/sodium hydroxide, lactic acid/lactate, monosodium phosphate/disodium phosphate, monopotassium phosphate/dipotassium phosphate, bicarbonate/carbonate, potassium diphthalate/acid, or boric acid/sodium borate.

15. Viscous liquid composition according to any one of claims 1 to 14, **characterized in that** the active ingredient is solubilized in the hydrating phase or in an organic solvent.

16. Viscous liquid composition according to claim 15, **characterized in that** the organic solvent is selected among vegetable oils, mineral oils, natural oils, synthetic oils, conventional lipophilic solvents, hydrophilics and non-toxic hydro-lipophilics.

17. Viscous liquid composition according to any one of claims 1 to 16, **characterized in that** it furthermore includes at least a second surface-active agent

18. Viscous liquid composition according to claim 17, **characterized in that** the second surface-active agent belongs to the class of ionic, nonionic and amphoteric surface-active agents.

19. Viscous liquid composition according to any one of claims 17 or 18, **characterized in that** the concentration of surface-active agents lies between 0 and 50% in mass in relation to the total mass of the excipients.

20. Viscous liquid composition according to any one of claims 1 to 19, **characterized in that** the active ingredient in the solid state is dispersed in the form of powder of a granulometry ranging between 1 µm and 1000 µm.

21. Viscous liquid composition according to claim 20, **characterized in that** the granulometry of the active ingredient lies between 1 µm and 250 µm.

22. Viscous liquid composition according to any one of claims 1 to 21, **characterized in that** it furthermore includes starch of rice, potato, corn, manioc, wheat and their derivatives.

23. Viscous liquid composition according to claim 22, **characterized in that** the starch concentration or modified starch lies between 0 and 50% in mass in relation to the total mass of the composition.

24. Viscous liquid composition according to any one of claims 1 to 23, **characterized in that** it includes at least a conservation, moistening, coloring, flavoring and/or sweetening agent.

25. Viscous liquid composition according to claim 24 **characterized in that** the concentration in preservatives lies between 0 and 10%.

26. Viscous liquid composition according to claim 24 **characterized in that** the concentration in moistening agents lies between 1 and 30%.

27. Viscous liquid composition according to claim 24, **characterized in that** the concentration in dyes lies between 0 and 5%.

28. Viscous liquid composition according to claim 24, **characterized in that** the concentration in sweetening substances lies between 0 and 30%.

29. Viscous liquid composition according to any one of claims 24 or 28, **characterized in that** the sweetening substances are natural or synthetic chosen among saccharose, aspartame, acesulfam, sodium cyclamate or sodium saccharinate.

30. Viscous liquid composition according to any one of claims 1 to 29 **characterized in that** it presents a viscosity ranging between 100 mPa and 500,000 mPa.

31. Viscous liquid composition according to any one of claims 1 to 30 **characterized in that** the release time of the active ingredient lies between 2 and 12 hours for the oral, nasal, and ocular routes.

32. Viscous liquid composition according to any one of claims 1 to 31 **characterized in that** the release time of the active ingredient is greater than 12 hours for the vaginal route.

33. Viscous liquid composition according to any one of claims 1 to 32, **characterized in that** the active ingredient belongs to a therapeutic class selected among: analgesics, anti-inflammatories, antispasmodics, cytotoxics, antibiotics, antifungals, disinfectants, anti-parasitics, hormones, antivirals, anti-migraine agents, antiallergics, analeptic respiratory agents, spermicides, anti-hemorrhoidal agents, vasoconstrictors, vasodilators, antipruritics, uterorelaxants, antiglaucoma agents, mydriatics and antiasthmatics.

## Patentansprüche

1. Viskose flüssige Zusammensetzung mit verstärkter Bioadhäsionsfähigkeit für eine örtliche Anwendung in Pastenform mit verlängerter Freisetzung eines Wirkstoffs, **dadurch gekennzeichnet, dass** sie Folgendes umfasst:
(i) mindestens ein Bioadhäsionsvehikel, das zu der Familie der Carrageenane gehört und die Bildung einer Matrixschicht mit verstärkter Bioadhäsionsfähigkeit durch Erzeugung von Komplexverbindungen des Bioadhäsionsvehikels und eines der Bestandteile von lokalen Sekreten oder der Schleimhaut *in situ* ermöglicht,
(ii) ein Medium zur Hydratation des Matrixmittels,
(iii) mindestens einen Wirkstoff,
und (iiii) mindestens eines grenzflächenaktiven Stoffs der Familie der Phospholipide, die das Verstärken der Bioadhäsionseigenschaften von Carrageenanen ermöglicht.

2. Viskose flüssige Zusammensetzung nach Anspruch 1, **dadurch gekennzeichnet, dass** die Phospholipide aus den Phosphatidylcholinen ausgewählt sind.

3. Viskose flüssige Zusammensetzung nach Anspruch 2, **dadurch gekennzeichnet, dass** die Phospholipide aus den Phosphatidylserinen ausgewählt sind.

4. Viskose flüssige Zusammensetzung nach Anspruch 1, **dadurch gekennzeichnet, dass** das Carrageenan aus den lambda- und iota-Carrageenanen ausgewählt ist.

5. Viskose flüssige Zusammensetzung nach Anspruch 1, **dadurch gekennzeichnet, dass** das Carrageenan in einer Konzentration zwischen 0,5 Gew.-% und 30 Gew.-%, bezogen auf das Gesamtgewicht der Zusammensetzung, vorliegt.

6. Viskose flüssige Zusammensetzung nach einem der Ansprüche 1 bis 5, **dadurch gekennzeichnet, dass** das Medium zur Hydratation des Matrixmittels eine wässrige oder hydroalkoholische Lösung ist.

7. Viskose flüssige Zusammensetzung nach Anspruch 6, **dadurch gekennzeichnet, dass** die hydroalkoholische Phase Ethanol oder Isopropylalkohol umfasst.

8. Viskose flüssige Zusammensetzung nach einem der Ansprüche 6 oder 7, **dadurch gekennzeichnet, dass** der Anteil an alkoholischer Phase zwischen 10 Gel.-% und 90 Gew.-%, bezogen auf das Gesamtvolumen der hydratisierenden Phase, ausmacht.

9. Viskose flüssige Zusammensetzung nach einem der Ansprüche 1 bis 8, **dadurch gekennzeichnet, dass** sie außerdem Alkali- oder Erdalkaliionen, vorzugsweise von Natrium oder Kalium, umfasst.

10. Viskose flüssige Zusammensetzung nach Anspruch 9, **dadurch gekennzeichnet, dass** der Anteil an Alkali- oder Erdalkaliionen von 0 bis 50 Gew.-%, bezogen auf das Gesamtgewicht der Zusammensetzung, variiert.

11. Viskose flüssige Zusammensetzung nach einem der Ansprüche 9 oder 10, **dadurch gekennzeichnet, dass** das Alkali- oder Erdalkaliion in der Form eines Hydroxids oder eines Salzes von Salzsäure, Schwefelsäure, Salpetersäure, Phosphorsäure, Citronensäure und/oder eines Derivats eingeführt wird.

12. Viskose flüssige Zusammensetzung nach einem der Ansprüche 1 bis 11, **dadurch gekennzeichnet, dass** die wässrige Phase des Mediums zur Hydratation eine Pufferlösung ist.

13. Viskose flüssige Zusammensetzung nach Anspruch 12, **dadurch gekennzeichnet, dass** der pH-Wert der Pufferlösung von 2 bis 12 variiert.

14. Viskose flüssige Zusammensetzung nach einem der Ansprüche 12 oder 13, **dadurch gekennzeichnet, dass** die Pufferlösung aus den Paaren Salzsäure/Natriumchlorid, Salzsäure/Kaliumphthalat, Salzsäure/Glycin, Citronensäure/Citrate, Citronensäure/Natriumhydroxid, Milchsäure/Lactat, Mononatriumphosphat/Dinatriumphosphat, Monokaliumphosphat/Dikaliumphosphat, Hydrogencarbonat/-Carbonat, Kaliumphthalat/Säure oder Borsäure/Natriumborat besteht.

15. Viskose flüssige Zusammensetzung nach einem der Ansprüche 1 bis 14, **dadurch gekennzeichnet, dass** der Wirkstoff in der hydratisierenden Phase oder in einem organischen Lösemittel solubilisiert wird.

16. Viskose flüssige Zusammensetzung nach Anspruch 15, **dadurch gekennzeichnet, dass** das organische Lösemittel aus pflanzlichen Ölen, Mineralölen, natürlichen Ölen, synthetischen Ölen, herkömmlichen lipophilen, hydrophilen und hydrolipophilen, nicht toxischen Lösemitteln ausgewählt ist.

17. Viskose flüssige Zusammensetzung nach einem der Ansprüche 1 bis 16, **dadurch gekennzeichnet, dass** sie außerdem mindestens einen zweiten grenzflächenaktiven Stoff umfasst.

18. Viskose flüssige Zusammensetzung nach Anspruch 17, **dadurch gekennzeichnet, dass** der zweite grenzflächenaktive Stoff zu der Klasse der ionischen, nichtionischen und amphoteren grenzflächenaktiven Stoffe gehört.

19. Viskose flüssige Zusammensetzung nach einem der Ansprüche 17 oder 18, **dadurch gekennzeichnet, dass** die Konzentration von grenzflächenaktiven Stoffen zwischen 0 und 50 Gew.-%, bezogen auf das Gesamtgewicht an Vehikeln, ausmacht.

20. Viskose flüssige Zusammensetzung nach einem der Ansprüche 1 bis 19, **dadurch gekennzeichnet, dass** der Wirkstoff im festen Zustand in der Form von Pulver mit einer Korngrößenverteilung zwischen 1 µm und 1000 µm dispergiert wird.

21. Viskose flüssige Zusammensetzung nach Anspruch 20, **dadurch gekennzeichnet, dass** die Korngrößenverteilung des Wirkstoffs zwischen 1 µm und 250 µm liegt.

22. Viskose flüssige Zusammensetzung nach einem der Ansprüche 1 bis 21, **dadurch gekennzeichnet, dass** sie außerdem Reis-, Kartoffel-, Mais-, Maniok-, Weizenstärke und deren Derivate umfasst.

23. Viskose flüssige Zusammensetzung nach Anspruch 22, **dadurch gekennzeichnet, dass** die Konzentration von Stärke oder modifizierter Stärke zwischen 0 und 50 Gew.-%, bezogen auf das Gesamtgewicht der Zusammensetzung, liegt.

24. Viskose flüssige Zusammensetzung nach einem der Ansprüche 1 bis 23, **dadurch gekennzeichnet, dass** sie mindestens ein Konservierungs-, Benetzungs-, Färbe-, Aroma- und/oder Süßungsmittel umfasst.

25. Viskose flüssige Zusammensetzung nach Anspruch 24, **dadurch gekennzeichnet, dass** die Konzentration von Konservierungsstoffen zwischen 0 und 10% liegt.

26. Viskose flüssige Zusammensetzung nach Anspruch 24, **dadurch gekennzeichnet, dass** die Konzentration von Benetzungsmitteln zwischen 1 und 30% liegt.

27. Viskose flüssige Zusammensetzung nach Anspruch 24, **dadurch gekennzeichnet, dass** die Konzentration von Farbstoffen zwischen 0 und 5% liegt.

28. Viskose flüssige Zusammensetzung nach Anspruch 24, **dadurch gekennzeichnet, dass** die Konzentration von Süßstoffen zwischen 0 und 30% liegt.

29. Viskose flüssige Zusammensetzung nach einem der Ansprüche 24 oder 28, **dadurch gekennzeichnet, dass** die Süßstoffe natürlich oder synthetisch sind und aus Saccharose, Aspartam, Acesulfam, Natriumcyclamat oder Natriumsaccharinat ausgewählt sind.

30. Viskose flüssige Zusammensetzung nach einem der Ansprüche 1 bis 29, **dadurch gekennzeichnet, dass** sie einen Viskosität zwischen 100 mPa und 500.000 mPa aufweist.

31. Viskose flüssige Zusammensetzung nach einem der Ansprüche 1 bis 30, **dadurch gekennzeichnet, dass** die Zeit der Freisetzung des Wirkstoffs bei der bukkalen, nasalen und okularen Route zwischen 2 und 12 Stunden liegt.

32. Viskose flüssige Zusammensetzung nach einem der Ansprüche 1 bis 31, **dadurch gekennzeichnet, dass** die Zeit der Freisetzung des Wirkstoffs bei der vaginalen Route mehr als 12 Stunden beträgt.

33. Viskose flüssige Zusammensetzung nach einem der Ansprüche 1 bis 32, **dadurch gekennzeichnet, dass** der Wirkstoff zu einer Therapeutikaklasse gehört und aus den Analgetika, den Antiphlogistika, den Antispastika, den Zytotoxika, den Antibiotika, den Antimykotika, den Antiseptika, den Antiparasitika, den Hormonen, den antiviralen Substanzen, den Migränemitteln, den Antiallergika, den Atemanaleptika, den Spermiziden, den Antihämorrhagika, den Vasokonstriktoren, den Vasodilatatoren, den Antipruritika, den Uterorelaxantien, den Antiglaukomatika, den Mydriatika, den Antiasmathika ausgewählt ist.
